# EUROPÄISCHE PATENTANMELDUNG

(11) **EP 4 166 062 A1**
(43) Veröffentlichungstag der Anmeldung: **19.04.2023**
(21) Anmeldenummer: 22201200.7
(22) Anmeldetag: 12.10.2022
(51) Int. Cl.: A61B 1/227, A61B 1/06, A61B 1/00

(54) **OTOSKOP**

(30) Priorität: 18.10.2021 DE 102021126955
(71) Anmelder: Heine Optotechnik GmbH & Co KG, 82205 Gilching (DE)
(72) Erfinder: KABBECK, Bernd, 82205 Gilching (DE); MARTIN, Timo, 82205 Gilching (DE); MICHEL, Felix, 82205 Gilching (DE)
(74) Vertreter: Flach Bauer & Partner Patentanwälte mbB

(57) **Zusammenfassung**

Ein Otoskop (10) hat einen Kopf (14), eine Halterung (16) für einen Ohrtrichter, eine primäre Beleuchtungseinrichtung (18) und eine sekundäre Beleuchtungseinrichtung (20). Die primäre Beleuchtungseinrichtung (18) ist für eine Untersuchung des Ohres ausgebildet und die sekundäre Beleuchtungseinrichtung (20) ist für eine Untersuchung eines vom Ohr verschiedenen Körperteils ausgebildet.

## Beschreibung

Die Erfindung betrifft ein Otoskop.

Otoskope zur Untersuchung des Ohres, insbesondere des Trommelfells, sind seit über 90 Jahren bekannt. Üblicherweise weisen solche Otoskope eine Halterung für einen wechselbaren Ohrtrichter sowie eine Optik mit Vergrößerung auf, deren Strahlengang durch eine zentrale Öffnung des Ohrtrichters gerichtet ist. Auf diese Weise kann eine Untersuchung des Trommelfells oder anderer Teile des Ohres erfolgen.

Zudem ist es bekannt, Otoskope mit einer Beleuchtungseinrichtung auszustatten, um den Arbeitsbereich vor der Halterung für den Ohrtrichter optimal für die Untersuchung des Ohrs beleuchten zu können.

Untersuchungen eines anderen Körperteils sind mit einem Otoskop jedoch nicht ohne Gefahren möglich.

Es ist daher Aufgabe der Erfindung, ein Otoskop bereitzustellen, dass eine Untersuchung anderer Körperteile außer dem Ohr ermöglicht. Die Aufgabe wird gelöst durch ein Otoskop mit einem Kopf, einer Halterung für einen Ohrtrichter, einer primären Beleuchtungseinrichtung und einer sekundären Beleuchtungseinrichtung. Die primäre Beleuchtungseinrichtung ist für eine Untersuchung des Ohres ausgebildet und die sekundäre Beleuchtungseinrichtung ist für eine Untersuchung eines vom Ohr verschiedenen Körperteils ausgebildet.

Aufgrund der sekundären Beleuchtungseinrichtung, die zur Untersuchung eines vom Ohr unterschiedlichen Körperteils ausgebildet und optimiert ist, sind Untersuchungen anderer Körperteile außer dem Ohr, beispielsweise des Auges, der Haut und/oder des Mund-Rachen-Raums, nun gefahrlos möglich.

Die spezielle Ausbildung zur Untersuchung von Körperteilen der primären und sekundären Beleuchtungseinrichtung kann sich auf einen beleuchteten Arbeitsbereich beziehen und/oder auf Lichteigenschaften, wie Wellenlänge, Farbtemperatur, Polarisation, Strahlgeometrie, maximale Helligkeit und dergleichen.

Insbesondere erstrecken sich die Strahlungsrichtungen der primären und der sekundären Beleuchtungseinrichtung von der Patientenseite des Kopfes aus in Richtung Patient.

Zum Beispiel verläuft der Strahlengang der sekundären Beleuchtungseinrichtung nicht durch eine zentrale Öffnung der Halterung und/oder verläuft vollständig außerhalb der Halterung.

Zum Beispiel ist die sekundäre Beleuchtungseinrichtung vollständig außerhalb der Halterung und/oder dem Ohrtrichter angeordnet.

Beispielsweise geht die Halterung vom Kopf aus und weist eine zentrale Öffnung auf.

In einem Aspekt definiert das Otoskop einen primären Arbeitsbereich und die primäre Beleuchtungseinrichtung ist dazu ausgebildet, den primären Arbeitsbereich zu beleuchten, insbesondere wobei die sekundäre Beleuchtungseinrichtung dazu ausgebildet ist, einen sekundären Arbeitsbereich zu beleuchten, der sich vom primären Arbeitsbereich unterscheidet. Dadurch sind verschiedene Arbeitsbereiche für verschiedene zu untersuchende Körperteile definiert und die Beleuchtungseinrichtungen sind somit besser für das jeweils zu untersuchende Körperteil ausgebildet.

Insbesondere sind der primäre und der sekundäre Arbeitsbereich vollständig unterschiedlich. Beispielsweise erstreckt sich der primäre Arbeitsbereich 1 cm bis 3 cm vor der zentralen Öffnung der Halterung.

Der primäre Arbeitsbereich kann axial vor der zentralen Öffnung angeordnet sein.

In einer Ausgestaltung weist das Otoskop eine Optik mit wenigstens einer Linse und/oder einer Kamera auf, wobei der Strahlengang der Optik durch die zentrale Öffnung der Halterung verläuft, insbesondere wobei der primäre Arbeitsbereich einem Bereich um den Fokus der Optik mit axialer Länge der Schärfentiefe der Optik entspricht, wodurch eine einfache Untersuchung des Ohres ermöglicht wird.

Die Ausdehnung des primären Arbeitsbereiches kann in radialer Richtung bezüglich der zentralen Öffnung so groß wie die zentrale Öffnung sein.

Zur einfachen und sicheren Handhabung kann sich die Halterung trichterförmig vom Kopf aus erstrecken und/oder an ihrer Außenseite ein Befestigungsmittel zur Befestigung eines Ohrtrichters aufweisen.

In einer Ausführungsform emittiert die primäre Beleuchtungseinrichtung Licht mit primären Lichteigenschaften und die sekundäre Beleuchtungseinrichtung emittiert Licht mit zumindest teilweise anderen Lichteigenschaften als die primären Lichteigenschaften, insbesondere wobei die Lichteigenschaften die Wellenlänge, die Farbtemperatur, die maximale Helligkeit, die Strahlgeometrie und/oder die Polarisation umfassen. Auf diese Weise können auch die Lichteigenschaften entsprechend dem zu untersuchenden Körperteil ausgebildet sein, wodurch die Qualität der Beleuchtung für den jeweiligen Verwendungszweck optimiert ist.

Beispielsweise hat das von der primären Beleuchtungseinrichtung emittierte Licht eine Wellenlänge von 200 nm bis 1000 nm, insbesondere von 380 nm bis 780 nm, eine Farbtemperatur von 3000 K bis 4500 K, eine maximale Helligkeit von 5 Im bis 20 lm und/oder keine Polarisation, insbesondere wobei die sekundäre Beleuchtungseinrichtung Licht mit einer Wellenlänge, einer Farbtemperatur und/oder einer Helligkeit zumindest teilweise außerhalb des jeweiligen Wertebereichs der primären Beleuchtungseinrichtung und/oder polarisiertes Licht emittiert. Auf diese Weise sind Anpassungen der Beleuchtung an die jeweils zu untersuchenden Körperteile möglich.

Zur weiteren Optimierung der Beleuchtung kann das von der primären Beleuchtungseinrichtung emittierte Licht eine Strahlgeometrie haben, die von der Strahlgeometrie des von der sekundären Beleuchtungseinrichtung emittierten Lichtes unterschiedlich ist.

In einem weiteren Aspekt hat der Kopf eine Griffseite, wobei die sekundäre Beleuchtungseinrichtung auf der von der Griffseite abgewandten Seite der Halterung am Kopf angeordnet ist. Durch diese Anordnung ist es für den Benutzer nicht notwendig, das Otoskop umzugreifen, wenn ein anderes Körperteil als das Ohr untersucht werden soll.

Zur weiteren Anpassung der Lichteigenschaften kann die sekundäre Beleuchtungseinrichtung wenigstens eine sekundäre Lichtquelle und ein optisches Element, insbesondere eine Linse, eine Blende und/oder einen Filter aufweisen.

Das optische Element ist insbesondere ein von der sekundären Lichtquelle separates Element.

Das optische Element kann eine Kugellinse sein.

Insbesondere verläuft der Strahlengang der sekundären Lichtquelle nicht durch die zentrale Öffnung der Halterung und/oder verläuft vollständig außerhalb der Halterung.

In einer Ausführungsform ist eines des wenigstens einen optischen Elementes eine Linse und/oder eine Blende, die im Strahlengang der sekundären Lichtquelle angeordnet ist, insbesondere wobei die Linse bzw. die Blende relativ zur sekundären Lichtquelle entlang der optischen Achse der sekundären Beleuchtungseinrichtung bewegbar ist. Dadurch lassen sich die Lichteigenschaften der zweiten Beleuchtungseinrichtung einfach verändern.

In einer Ausgestaltung weist die sekundäre Beleuchtungseinrichtung einen beweglichen Träger auf, in dem verschiedene optische Elemente der sekundären Beleuchtungseinrichtung, insbesondere verschiedene optische Elemente des gleichen Typs, angeordnet und/oder ausgebildet sind, wobei der bewegliche Träger derart durch den Strahlengang der sekundären Lichtquelle verläuft und bewegt werden kann, dass dasjenige der optischen Elemente des Trägers gewechselt werden kann, das sich im Strahlengang befindet. Mittels der verschiedenen optischen Elemente des Trägers können die Lichteigenschaften der sekundären Beleuchtungseinrichtung über einen äußerst weiten Bereich angepasst werden.

Beispielweise ist der Träger ein Ring oder Ringabschnitt, wobei der Ring oder Ringabschnitt konzentrisch zur zentralen Öffnung der Halterung ausgebildet ist und um die Achse der zentralen Öffnung herum drehbar ausgebildet ist, wobei die optischen Achsen der optischen Elemente parallel zur Achse der zentralen Öffnung verlaufen, wodurch eine besonders große Anzahl an verschiedenen optischen Elementen auf dem Träger ermöglicht wird.

Alternativ oder zusätzlich ist der Träger ein Ring oder Ringabschnitt, wobei der Ring oder Ringabschnitt um eine Drehachse herum drehbar ausgebildet ist, die senkrecht zur Achse der zentralen Öffnung verläuft, wobei die optischen Achsen der optischen Elemente senkrecht zur Drehachse verlaufen, wodurch der Träger platzsparend ausgebildet ist.

In einer Ausführungsform weist die sekundäre Beleuchtungseinrichtung mehrere sekundäre Lichtquellen und/oder mehrere Baugruppen mit wenigstens einer sekundären Lichtquelle und einem optischen Element auf, wobei die Lichtquellen oder Baugruppen Licht mit voneinander verschiedenen Lichteigenschaften emittieren und/oder wobei die Lichtquellen und/oder Baugruppen um die Halterung herum am Kopf angeordnet sind. Auf diese Weise können die Lichteigenschaften des von den verschiedenen Baugruppen emittierten Lichtes jeweils für die Untersuchung unterschiedlicher Körperteile optimiert werden.

Die Lichtquellen der verschiedenen Baugruppen können unterschiedlichen Typs sein, zum Beispiel LED oder Laserdioden.

Zur einfachen Handhabung kann das Otoskop einen Griff aufweisen, an dem der Kopf befestigt ist, insbesondere wobei am Griff zumindest ein Bedienelement vorgesehen ist, mit dem die primäre und/oder die sekundäre Beleuchtungseinrichtung steuerbar ist.

Weitere Merkmale und Vorteile der Erfindung ergeben sich aus der nachfolgenden Beschreibung sowie aus den beigefügten Zeichnungen, auf die Bezug genommen wird. In den Zeichnungen zeigen:
- Figur 1:: ein erfindungsgemäßes Otoskop in perspektivischer Ansicht von hinten,
- Figur 2:: das Otoskop gemäß Figur 1 in einer perspektivischen Ansicht von vorne auf den Kopf,
- Figur 3:: eine Schnittansicht durch das Otoskop gemäß Figur 1,
- Figur 4:: eine Schnittansicht durch eine zweite Ausführungsform eines erfindungsgemäßen Otoskops,
- Figur 5:: eine Frontansicht eines Kopfes eines erfindungsgemäßen Otoskops gemäß einer dritten Ausführungsform,
- Figur 6:: einen Träger für optische Elemente des Otoskops gemäß Figur 5, und
- Figur 7:: eine Schnittansicht durch ein erfindungsgemäßes Otoskop gemäß einer vierten Ausführungsform.

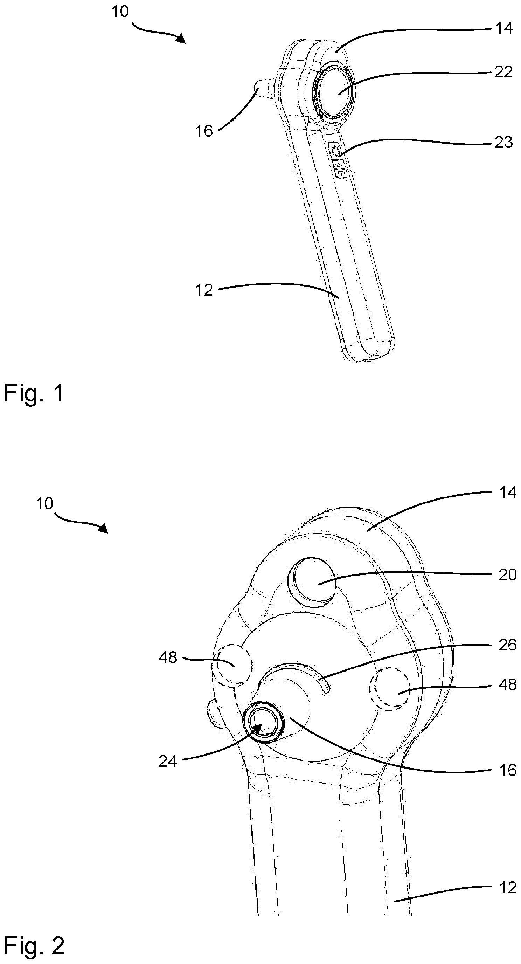

In Figur 1 ist ein Otoskop 10 in einer perspektivischen Ansicht von hinten dargestellt, also von derjenigen Seite, die bei der ordnungsgemäßen Verwendung dem Benutzer, beispielsweise einem Arzt, zugewandt ist.

Diese Seite wird daher im Rahmen dieser Offenbarung auch "Benutzerseite" genannt. Die dazu entgegengesetzte Seite wird "Patientenseite" genannt.

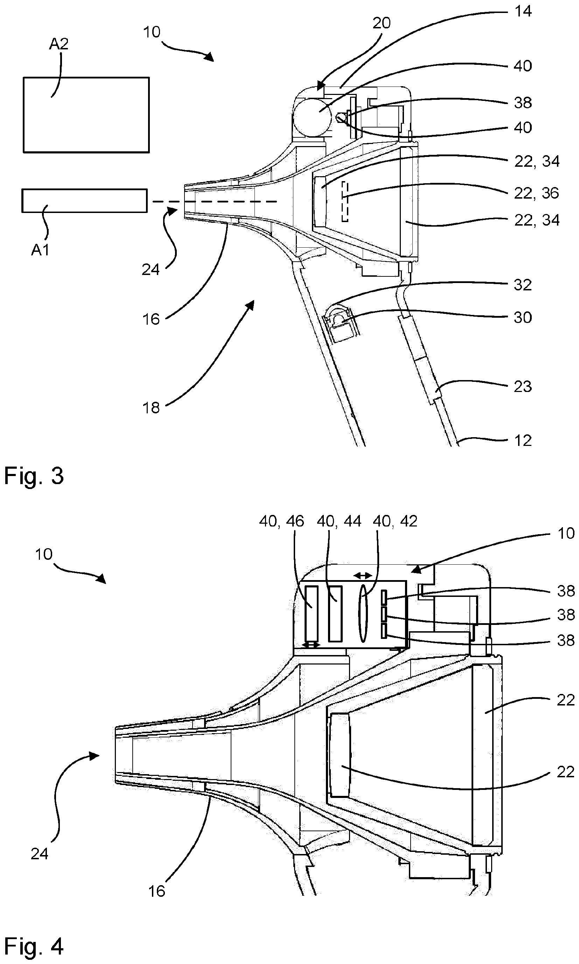

Das Otoskop 10 weist einen Griff 12, einen Kopf 14, eine Halterung 16 für einen Ohrtrichter, eine primäre Beleuchtungseinrichtung 18 (Fig. 3), eine sekundäre Beleuchtungseinrichtung (Fig. 2), eine Optik 22 sowie zwei Bedienelemente 23 auf.

Im Rahmen dieser Erfindung werden die Begriffe "primär" und "sekundär" zur Unterscheidung von verschiedenen Bauteilen verwendet und geben keine Mindestanzahl oder Hierarchien zwischen den Bauteilen an.

Die Bedienelemente 23 sind im gezeigten Ausführungsbeispiel am Griff 12 angeordnet und beispielsweise Taster.

Mittels der Bedienelemente 23 können die primäre Beleuchtungseinrichtung 18 und/oder die sekundäre Beleuchtungseinrichtung 20 bedient bzw. gesteuert werden.

In den Figuren 2 und 3 ist der Kopf 14 des Otoskops 10 in einer perspektivischen Ansicht von vorne bzw. in einer Schnittansicht dargestellt.

Die Halterung 16 ist am Kopf 14 angeordnet, beispielsweise erstreckt sie sich einstückig vom Kopf aus.

Die Halterung 16 hat eine Trichterform und verjüngt sich vom Kopf 14 ausgehend bis zu der vom Kopf 14 entferntesten Stelle, an der eine zentrale Öffnung 24 ausgebildet ist.

An der Außenseite weist die Halterung in an sich bekannter Weise ein Befestigungsmittel 26 zur Befestigung eines Ohrtrichters (nicht gezeigt) auf.

Die Halterung 16 ist doppelwandig ausgeführt, wie in Figur 3 zu sehen ist, wobei die beiden Wände der Halterung 16 konzentrisch um die Achse der zentralen Öffnung 24 verlaufen, jedoch mit verschiedenen Radien.

Im Bereich der zentralen Öffnung 24 entsteht somit ein Ringspalt zwischen den beiden Wänden.

Der Bereich zwischen den beiden Wänden und somit auch der Ringspalt stellen einen Teil der primären Beleuchtungseinrichtung 18 dar. Insbesondere entsprechen die doppelte Wand und der entstehende Ringspalt einer ringförmigen Blende.

Die primäre Beleuchtungseinrichtung 18 weist zudem eine primäre Lichtquelle 30 und optional ein optisches Element 32, im gezeigten Ausführungsbeispiel eine Linse auf.

Zudem kann die primäre Beleuchtungseinrichtung 18 ein Bündel von Glasfasern aufweisen, das in den Figuren zur Verbesserung der Übersicht nicht dargestellt ist. Das Glasfaserbündel verläuft von der primären Beleuchtungseinrichtung 18 bzw. dem optischen Element 32 zwischen den beiden Wänden der Halterung 16 zum Ringspalt.

Die primäre Lichtquelle 30 ist beispielsweise dazu ausgebildet, Licht mit bestimmten Lichteigenschaften zu emittieren. Zum Beispiel ist die primäre Lichtquelle 30 eine LED.

Die Lichteigenschaften können die Wellenlänge, beispielsweise die mittlere Wellenlänge, die Farbtemperatur, die maximale Helligkeit und/oder die Polarisation umfassen.

Die primäre Lichtquelle 30 und im Endeffekt damit die primäre Beleuchtungseinrichtung 18 emittieren Licht, das optimal für die Untersuchung des Ohres ist, zum Beispiel unpolarisiertes Licht mit einer Wellenlänge zwischen 200 nm und 1000 nm, insbesondere zwischen 380 nm und 780 nm, einer Farbtemperatur von 3000 K bis 4500 K, und einer maximalen Helligkeit von 5 Im bis 20 Im.

Das optische Element 32 ist im gezeigten Beispiel eine Linse, kann jedoch auch ein Filter, wie ein Wellenlängenfilter oder Polarisationsfilter sein. Auch der Ringspalt an der zentralen Öffnung ist als optisches Elemente 32 der primären Beleuchtungseinrichtung 18 anzusehen.

Die Optik 22 ist am Kopf 14 und zumindest teilweise im Kopf 14 ausgebildet und hat einen Strahlengang, der sich durch den Kopf 14 erstreckt, genauer gesagt von der Benutzerseite (in Figur 3 rechts) zur Patientenseite (in Figur 3 links). Der Strahlengang verläuft innerhalb der inneren Wand der Halterung 16.

Die Optik 22 weist im gezeigten Ausführungsbeispiel zwei Linsen 34 auf, die einen primären Arbeitsbereich A1 definieren, wobei eine der Linsen 34 im Kopf 14 und die andere der Linsen 34 an der Benutzerseite am Kopf 14 befestigt ist.

Der primäre Arbeitsbereich A1 entspricht beispielsweise einem Bereich in Axialrichtung der zentralen Öffnung 24 bzw. in Richtung des Strahlengangs der Optik 22 vor der zentralen Öffnung 24, der sich um den Fokus der Optik 22 herum erstreckt. Der primäre Arbeitsbereich A1 kann dabei eine axiale Länge haben, die der Schärfentiefe der Optik 22 entspricht. In radialer Richtung ist der primäre Arbeitsbereich A1 in etwa so groß wie die zentrale Öffnung 24.

Anstelle einer oder beider Linsen 34 kann in der Optik 22 auch eine Kamera 36 (in Figur 3 gestrichelt angedeutet) vorgesehen sein.

Der primäre Arbeitsbereich A1 wird bei einer Untersuchung des Ohres mittels der primären Beleuchtungseinrichtung 18 beleuchtet.

Beim Betrieb der primären Beleuchtungseinrichtung wird also Licht von der primären Lichtquelle 30 mit den Lichteigenschaften emittiert, passiert dann das eine oder die mehreren optischen Elemente 32, wodurch beispielsweise die Lichteigenschaften, wie die Polarisation, geändert werden können. Das Licht koppelt dann in die Glasfasern des Glasfaserbündels ein und läuft im Glasfaserbündel zwischen den beiden Wänden der Halterung 16 bis zur zentralen Öffnung 24, an der es schließlich aus dem Otoskop 10 austritt.

Beim Austritt durch die zentrale Öffnung 24 hat das Licht der primären Beleuchtungseinrichtung 18 primäre Lichteigenschaften, die auch die Strahlgeometrie, die in diesem Falle ringförmig ist, umfassen.

Auf diese Weise wird der primäre Arbeitsbereich A1 durch die primäre Beleuchtungseinrichtung 18 optimal ausgeleuchtet. Die primäre Beleuchtungseinrichtung 18 ist somit dazu ausgebildet, den primären Arbeitsbereich A1 zu beleuchten.

Der primäre Arbeitsbereich A1 erstreckt sich beispielsweise zwischen 1 cm bis 3 cm in Axialrichtung vor der zentralen Öffnung 24 der Halterung 16.

Die sekundäre Beleuchtungseinrichtung 20 ist ebenfalls im Kopf 14 des Otoskops 10 angeordnet.

Die sekundäre Beleuchtungseinrichtung 20 ist im Gegensatz zur primären Beleuchtungseinrichtung 18 zur Untersuchung eines vom Ohr verschiedenen Körperteils ausgebildet und optimiert.

Die sekundäre Beleuchtungseinrichtung 20 ist beispielsweise oberhalb der Optik 22 und der Halterung 16 angeordnet, d. h. auf der vom Griff 12 bzw. der Griffseite abgewandten Seite des Kopfes 14.

Die sekundäre Beleuchtungseinrichtung 20 ist somit vollständig außerhalb der Halterung 16 und/oder dem Ohrtrichter ausgebildet.

Die sekundäre Beleuchtungseinrichtung 20 beleuchtet einen sekundären Arbeitsbereich A2, der vom primären Arbeitsbereich A1 unterschiedlich oder teilweise unterschiedlich ist. Insbesondere bestehen keine Überlappungen zwischen dem primären Arbeitsbereich A1 und dem sekundären Arbeitsbereich A2 oder der Bereich der Überlappungen ist kleiner als 20% des Gesamtvolumens des primären Arbeitsbereiches A1.

Der sekundäre Arbeitsbereich A2 befindet sich vor der gleichen Seite des Kopfes 14 wie der primäre Arbeitsbereich A1, also auf der Patientenseite des Kopfes 14.

Entsprechend verlaufen die Strahlrichtungen der primären Beleuchtungseinrichtung 18 und der sekundären Beleuchtungseinrichtung 20 auf der gleichen Seite des Kopfes 14.

Der Strahlengang der sekundären Beleuchtungseinrichtung 20 verläuft vollständig außerhalb der Halterung 16, insbesondere nicht durch die zentrale Öffnung 24 der Halterung 16.

Die sekundäre Beleuchtungseinrichtung 20 ist beispielsweise in einem Hohlraum des Kopfes 14 ausgebildet und weist eine sekundäre Lichtquelle 38 sowie wenigstens ein optisches Element 40 auf.

Im gezeigten ersten Ausführungsbeispiel gemäß Figur 3 weist die sekundäre Beleuchtungseinrichtung 20 zwei optische Elemente 40 in Form von Linsen, beispielsweise Kugellinsen auf.

Die optischen Elemente 40 sind von der sekundären Lichtquelle 38 separate Elemente.

Denkbar ist auch die Verwendung einer einzelnen Kugellinse.

Die sekundäre Lichtquelle 38 emittiert Licht mit bestimmten Lichteigenschaften, wobei das Licht durch die optischen Elemente 40 verläuft, bevor das Licht den sekundären Arbeitsbereich A2 ausleuchtet. Die sekundäre Lichtquelle 38 ist beispielsweise eine LED oder ein Laser.

Insbesondere verläuft der Strahlengang der sekundären Lichtquelle 38 nicht durch die zentrale Öffnung 24 der Halterung 16 und/oder verläuft vollständig außerhalb der Halterung 16.

Die sekundären Lichteigenschaften des von der sekundären Beleuchtungseinrichtung 20 emittierten Lichtes unterscheiden sich zumindest teilweise von den primären Lichteigenschaften der primären Beleuchtungseinrichtung 18 im primären Arbeitsbereich A1. Insbesondere liegen die Werte der sekundären Lichteigenschaften zumindest teilweise außerhalb des jeweiligen Wertebereiches der primären Lichteigenschaften.

Beispielsweise ist das von der sekundären Beleuchtungseinrichtung 20 emittierte Licht zur Untersuchung des Auges ausgebildet und weist deswegen einen anderen Wellenlängenbereich, eine andere Farbtemperatur, eine andere maximale Helligkeit und/oder eine andere Polarisation auf.

Insbesondere ist die Strahlgeometrie des von der sekundären Beleuchtungseinrichtung 20 emittierten Lichtes anders als die ringförmige Strahlgeometrie der primären Beleuchtungseinrichtung 18.

Ein Benutzer, insbesondere ein Arzt, kann das Otoskop 10 in an sich bekannter Weise zur Untersuchung des Ohres verwenden, in dem ein Ohrtrichter auf die Halterung 16 aufgesetzt wird und in das Ohr des Patienten eingeführt wird. Hierzu wird die primäre Beleuchtungseinrichtung 18 aktiviert und der Benutzer untersucht das Ohr mithilfe der Optik 22.

Möchte der Benutzer nun ein anderes Körperteil, beispielsweise das Auge untersuchen, so deaktiviert der Benutzer die primäre Beleuchtungseinrichtung 18 und aktiviert die sekundäre Beleuchtungseinrichtung 20. Dies kann er mittels der Bedienelemente 23 tun.

Aufgrund der Ausbildung der sekundären Beleuchtungseinrichtung 20 für eine Untersuchung des Auges kann der Benutzer eine optimale Untersuchung ohne Instrumentenwechsel durchführen.

Die Figuren 4 bis 7 zeigen weitere Ausführungsformen des erfindungsgemäßen Otoskops, die im Wesentlichen der der ersten Ausführungsform entsprechen. Daher wird im Folgenden nur auf die Unterschiede eingegangen und gleiche und funktionsgleiche Teile sind mit denselben Bezugszeichen versehen.

In Figur 4 ist ein Schnitt ähnlich dem der Figur 3 durch den Kopf 14 des Otoskops 10 dargestellt.

In diesem zweiten Ausführungsbeispiel ist die sekundäre Beleuchtungseinrichtung 20 unterschiedlich ausgeführt.

Zum einen weist die sekundäre Beleuchtungseinrichtung 20 drei sekundäre Lichtquellen 38 auf, die insbesondere Licht mit unterschiedlichen Lichteigenschaften emittieren können oder sogar Lichtquellen unterschiedlichen Typs (LED, Laser) sind.

Zudem sind drei optische Elemente 40 vorgesehen, nämlich eine Linse 42, ein Filter 44 sowie eine Blende 46, die im Strahlengang der sekundären Lichtquellen 38 angeordnet sind.

Mittels der optischen Elemente 40 können die sekundären Lichteigenschaften des von der sekundären Beleuchtungseinrichtung 20 emittierten Lichtes weiter angepasst werden.

Eines oder mehrere der optischen Elemente 40, insbesondere die Blende 46 und/oder die Linse 42 können beweglich befestigt sein, sodass sie in Richtung des Strahlengangs relativ zur sekundären Lichtquelle 38 linear bewegt werden können, wie durch die Pfeile in Figur 4 angedeutet ist. Durch das Verschieben lässt sich die Strahlgeometrie der sekundären Beleuchtungseinrichtung 20 anpassen.

Denkbar ist, dass der Filter 44 ein Polarisationsfilter ist oder dass die Blende 46 eine Strichblende ist.

Die optischen Elemente 40 und zumindest eine sekundäre Lichtquelle 38 bilden eine Baugruppe 48. Denkbar ist, dass die sekundäre Beleuchtungseinrichtung 20 verschiedene dieser Baugruppen 48 aus einer sekundären Lichtquelle 38 und entsprechenden optischen Elementen 40 aufweist, um optimiertes Licht für verschiedene Untersuchungen anderer Körperteile als das Ohr bereitstellen zu können.

Beispielsweise beleuchtet jede der Baugruppen den sekundären Arbeitsbereich A2.

Die verschiedenen Baugruppen 48 können am Kopf 14 zumindest teilweise um die Halterung 16 angeordnet sein, wie in Figur 2 mittels der gestrichelten Kreise angedeutet ist.

Zum Beispiel kann das emittierte Licht einer der Baugruppen 48 für eine Untersuchung des Auges, das Licht einer anderen Baugruppe 48 für die Untersuchung des Mund-Rachen-Raums ausgebildet oder optimiert sein.

Insbesondere können die sekundären Lichtquellen 38 der verschiedenen Baugruppen 48 unterschiedlich sein. So kann eine Baugruppe 48 als sekundäre Lichtquelle 38 eine Laserdiode und eine strukturierte Blende aufweisen, um sogenanntes strukturiertes Licht im sekundären Arbeitsbereich A2 bereitzustellen.

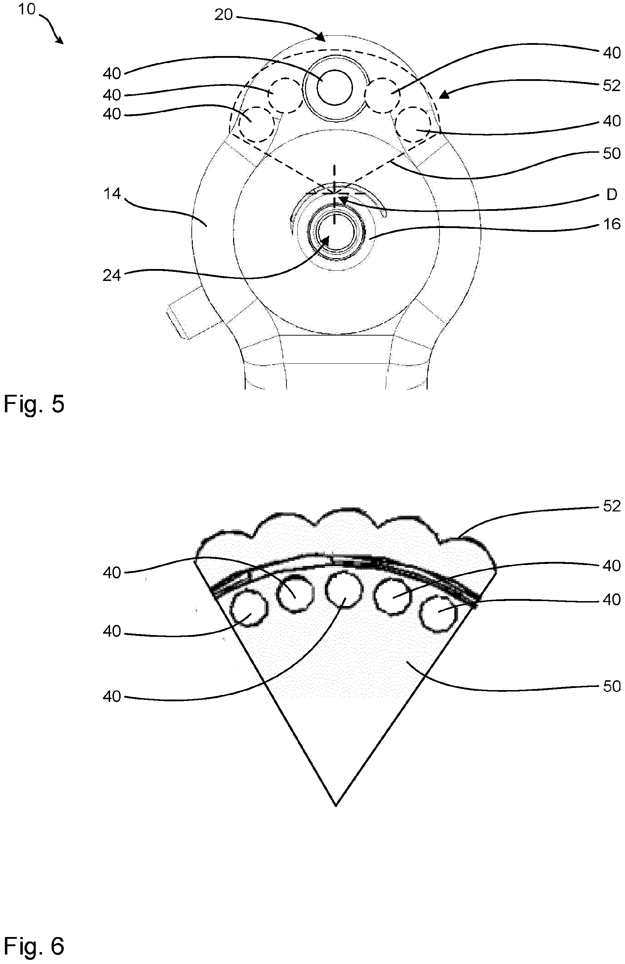

Die Figuren 5 und 6 zeigen eine dritte Ausführungsform des erfindungsgemäßen Otoskops 10.

Die dritte Ausführungsform entspricht im Wesentlichen der zweiten Ausführungsform gemäß Figur 4, wobei eines der optischen Elemente 40 der sekundären Beleuchtungseinrichtung 20 verändert werden kann.

Hierzu weist das Otoskop 10 im Kopf 14 einen Träger 50 auf, an oder in dem verschiedene optische Elemente 40 des gleichen Typs ausgebildet sind. Beispielsweise weist der Träger 50 verschiedene Linsen 42, verschiedene Filter 44 oder verschiedene Blenden 46 auf. In Figur 6 ist ein solcher Träger 50 beispielhaft dargestellt.

Der Träger 50 kann als Ring oder - wie dargestellt - als Ringabschnitt ausgeführt sein, wobei die optischen Elemente 40 in Umfangsrichtung nebeneinander angeordnet sind. Insbesondere weisen die optischen Elemente 40 den gleichen Abstand zum Mittelpunkt des Rings auf.

Die optischen Achsen der optischen Elemente 40 weisen dabei alle die gleiche Richtung, nämlich senkrecht zur Ebene des Trägers 50.

Die radial äußere Oberfläche des Trägers 50 kann eine Betätigungskontur 52 aufweisen, um den Träger 50 manuell verdrehen zu können.

Wie in Figur 5 angedeutet, verläuft der Träger 50 im Strahlengang der sekundären Lichtquelle 38, sodass eines der optischen Elemente 40 im Strahlengang angeordnet ist.

Der Träger 50 ist um eine Drehachse D drehbar im Kopf 14 gelagert, wobei die Betätigungskontur 52 des Trägers 50 zur manuellen Betätigung aus dem Kopf 14 hervorsteht. Die Drehachse verläuft dabei parallel zur Achse der zentralen Öffnung 24 der Halterung 16, insbesondere entspricht die Drehachse D der Achse der zentralen Öffnung 24.

Durch Drehung des Trägers 50 kann dasjenige optische Element 40, das sich im Strahlengang der sekundären Lichtquelle 38 befindet, gewechselt werden. Auf diese Weise können die Lichteigenschaften des von der sekundären Beleuchtungseinrichtung 20 emittierten Lichtes geändert werden, um es an die gewünschte Behandlungssituation bzw. das zu untersuchende Körperteil anzupassen.

Alternativ oder zusätzlich zur manuellen Betätigung ist auch eine automatische, beispielsweise eine elektrisch betriebene Drehung des Trägers 50 denkbar.

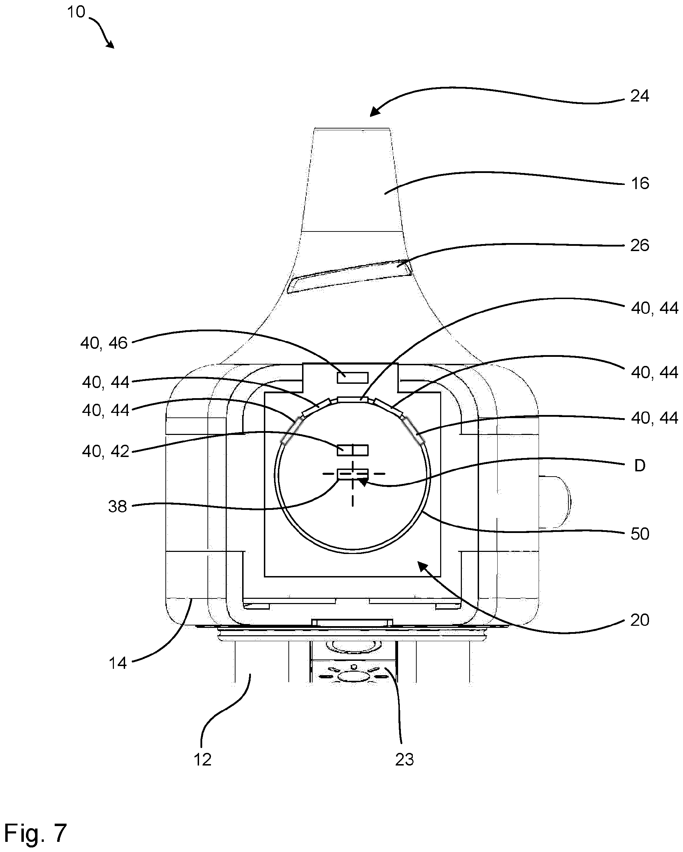

In Figur 7 ist eine vierte Ausführungsform des erfindungsgemäßen Otoskops 10 dargestellt. Figur 7 zeigt einen horizontalen Schnitt durch den Kopf 14 des Otoskops 10 oberhalb der Halterung 16 bzw. der Optik 22 durch die sekundäre Beleuchtungseinrichtung 20.

Die sekundäre Beleuchtungseinrichtung 20 der vierten Ausführungsform entspricht im Wesentlichen der der zweiten Ausführungsform gemäß Figur 4.

Zudem ist, ähnlich wie zur dritten Ausführungsform beschrieben, in der vierten Ausführungsform ein Träger 50 vorgesehen, auf dem verschiedene optische Elemente 40 des gleichen Typs angeordnet sind, um die optischen Elemente im Strahlengang zu wechseln.

In der vierten Ausführungsform ist der Träger 50 ein Ring, könnte aber ebenso gut ein Ringabschnitt oder ein ebener Schieber sein.

Der Träger 50 ist drehbar gegenüber dem Kopf 14 um eine Drehachse D ausgeführt, die sich senkrecht zum Strahlengang bzw. zur Achse der zentralen Öffnung 24 erstreckt, zum Beispiel nach oben. Der Träger 50 ist um die sekundäre Lichtquelle 38 und gegebenenfalls auch um ein optisches Element 40 herum angeordnet. Die optischen Achsen der optischen Elemente 40 des Trägers 50 weisen dabei in radialer Richtung des Trägers 50.

Durch Drehung des Trägers 50 kann auch in dieser Ausführungsform das optische Element 40, welches im Strahlengang angeordnet ist, gewechselt werden. Auch hierdurch können die Lichteigenschaften der sekundären Beleuchtungseinrichtung 20 angepasst werden.

Ist der Träger 50 ein Schieber, kann eine lineare Bewegung des Trägers senkrecht zum Strahlengang erfolgen.

Auch in dieser Ausführungsform kann die Drehung oder Bewegung des Trägers 50 entweder manuell oder automatisch, beispielsweise mittels eines Elektromotors erfolgen.

Denkbar ist, dass die sekundäre Beleuchtungseinrichtung 20, insbesondere die Lichteigenschaften, mithilfe der Bedienelemente 23 ausgewählt oder gesteuert werden können.

Die verschiedenen Merkmale der verschiedenen Ausführungsformen können miteinander kombiniert werden. Insbesondere ist es denkbar, dass die zweite, dritte und vierte Ausführungsform (Figuren 4, 5 und 7) miteinander kombiniert werden, wobei ein optisches Element 40, zum Beispiel die Blende 46, mittels eines Trägers 50, wie zur dritten Ausführungsform (Figur 5) beschrieben, gewechselt werden kann und ein weiteres optisches Element 40, zum Beispiel der Filter 44 oder die Linse 42, mittels eines Trägers 50, wie zur vierten Ausführungsform (Figur 7) beschrieben, gewechselt werden kann.

Denkbar ist es auch, dass mehrere Träger 50 des gleichen Typs zum Einsatz kommen, um verschiedene optische Elemente 40 wechseln zu können.

## Patentansprüche

1. Otoskop mit einem Kopf (14), einer Halterung (16) für einen Ohrtrichter, einer primären Beleuchtungseinrichtung (18) und einer sekundären Beleuchtungseinrichtung (20), wobei die primäre Beleuchtungseinrichtung (18) für eine Untersuchung des Ohres ausgebildet ist und die sekundäre Beleuchtungseinrichtung (20) für eine Untersuchung eines vom Ohr verschiedenen Körperteils ausgebildet ist.

2. Otoskop nach Anspruch 1, **dadurch gekennzeichnet, dass** das Otoskop (10) einen primären Arbeitsbereich (A1) definiert und die primäre Beleuchtungseinrichtung (18) dazu ausgebildet ist, den primären Arbeitsbereich (A1) zu beleuchten, insbesondere wobei die sekundäre Beleuchtungseinrichtung (20) dazu ausgebildet ist, einen sekundären Arbeitsbereich (A2) zu beleuchten, der sich vom primären Arbeitsbereich (A1) unterscheidet.

3. Otoskop nach Anspruch 1 oder 2, **dadurch gekennzeichnet, dass** das Otoskop (10) eine Optik (22) mit wenigstens einer Linse (34) und/oder einer Kamera (36) aufweist, wobei der Strahlengang der Optik (22) durch die zentrale Öffnung (24) der Halterung (16) verläuft, insbesondere wobei der primäre Arbeitsbereich (A1) einem Bereich um den Fokus der Optik (22) mit axialer Länge der Schärfentiefe der Optik (22) entspricht.

4. Otoskop nach einem der vorhergehenden Ansprüche, **dadurch gekennzeichnet, dass** sich die Halterung (16) trichterförmig vom Kopf (14) aus erstreckt und/oder an ihrer Außenseite ein Befestigungsmittel (26) zur Befestigung eines Ohrtrichters aufweist.

5. Otoskop nach einem der vorhergehenden Ansprüche, **dadurch gekennzeichnet, dass** die primäre Beleuchtungseinrichtung (18) Licht mit primären Lichteigenschaften emittiert und die sekundäre Beleuchtungseinrichtung (20) Licht mit zumindest teilweise anderen Lichteigenschaften als die primären Lichteigenschaften emittiert, insbesondere wobei die Lichteigenschaften die Wellenlänge, die Farbtemperatur, die maximale Helligkeit, die Strahlgeometrie und/oder die Polarisation umfassen.

6. Otoskop nach einem der vorhergehenden Ansprüche, **dadurch gekennzeichnet, dass** das von der primären Beleuchtungseinrichtung (18) emittierte Licht eine Wellenlänge von 200 nm bis 1000 nm, insbesondere von 380 nm bis 780 nm, eine Farbtemperatur von 3000 K bis 4500 K, eine maximale Helligkeit von 5 Im bis 15 Im und/oder keine Polarisation hat, insbesondere wobei die sekundäre Beleuchtungseinrichtung (20) Licht mit einer Wellenlänge, einer Farbtemperatur und/oder einer Helligkeit zumindest teilweise außerhalb des jeweiligen Wertebereichs der primären Beleuchtungseinrichtung und/oder polarisiertes Licht emittiert.

7. Otoskop nach einem der vorhergehenden Ansprüche, **dadurch gekennzeichnet, dass** das von der primären Beleuchtungseinrichtung (18) emittierte Licht eine Strahlgeometrie hat, die von der Strahlgeometrie des von der sekundären Beleuchtungseinrichtung (20) emittierten Lichtes unterschiedlich ist.

8. Otoskop nach einem der vorhergehenden Ansprüche, **dadurch gekennzeichnet, dass** der Kopf (14) eine Griffseite hat, wobei die sekundäre Beleuchtungseinrichtung (20) auf der von der Griffseite abgewandten Seite der Halterung (16) am Kopf (14) angeordnet ist.

9. Otoskop nach einem der vorhergehenden Ansprüche, **dadurch gekennzeichnet, dass** die sekundäre Beleuchtungseinrichtung (20) wenigstens eine sekundäre Lichtquelle (38) und ein optisches Element (40), insbesondere eine Linse (42), eine Blende (46) und/oder einen Filter (44) aufweist.

10. Otoskop nach Anspruch 9, **dadurch gekennzeichnet, dass** eines des wenigstens einen optischen Elementes (40) eine Linse (34) und/oder eine Blende (46) ist, die im Strahlengang der sekundären Lichtquelle (38) angeordnet ist, insbesondere wobei die Linse (34) bzw. die Blende (46) relativ zur sekundären Lichtquelle (38) entlang der optischen Achse der sekundären Beleuchtungseinrichtung (20) bewegbar ist.

11. Otoskop nach Anspruch 9 oder 10, **dadurch gekennzeichnet, dass** die sekundäre Beleuchtungseinrichtung (20) einen beweglichen Träger (50) aufweist, in dem verschiedene optische Elemente (40) der sekundären Beleuchtungseinrichtung (20), insbesondere verschiedene optische Elemente (40) des gleichen Typs, angeordnet und/oder ausgebildet sind, wobei der bewegliche Träger (50) derart durch den Strahlengang der sekundären Lichtquelle (38) verläuft und bewegt werden kann, dass dasjenige der optischen Elemente (40) des Trägers (50) gewechselt werden kann, das sich im Strahlengang befindet.

12. Otoskop nach Anspruch 11, **dadurch gekennzeichnet, dass** der Träger (50) ein Ring oder Ringabschnitt ist, wobei der Ring oder Ringabschnitt konzentrisch zur zentralen Öffnung (24) der Halterung (16) ausgebildet ist und um die Achse der zentralen Öffnung (24) herum drehbar ausgebildet ist, wobei die optischen Achsen der optischen Elemente (40) parallel zur Achse der zentralen Öffnung (24) verlaufen.

13. Otoskop nach Anspruch 11 oder 12, **dadurch gekennzeichnet, dass** der Träger (50) ein Ring oder Ringabschnitt ist, wobei der Ring oder Ringabschnitt um eine Drehachse (D) herum drehbar ausgebildet ist, die senkrecht zur Achse der zentralen Öffnung (24) verläuft, wobei die optischen Achsen der optischen Elemente (40) senkrecht zur Drehachse (D) verlaufen.

14. Otoskop nach einem der vorhergehenden Ansprüche, **dadurch gekennzeichnet, dass** die sekundäre Beleuchtungseinrichtung (20) mehrere sekundäre Lichtquellen (38) und/oder mehrere Baugruppen (48) mit wenigstens einer sekundären Lichtquelle (38) und einem optischen Element (40) aufweist, wobei die sekundären Lichtquellen (38) oder Baugruppen (48) Licht mit voneinander verschiedenen Lichteigenschaften emittieren und/oder wobei die sekundären Lichtquellen (38) und/oder Baugruppen (48) um die Halterung (16) herum am Kopf (14) angeordnet sind.

15. Otoskop nach einem der vorhergehenden Ansprüche, **dadurch gekennzeichnet, dass** das Otoskop (10) einen Griff (12) aufweist, an dem der Kopf (14) befestigt ist, insbesondere wobei am Griff (12) zumindest ein Bedienelement (23) vorgesehen ist, dass dazu ausgebildet ist, die primäre Beleuchtungseinrichtung (18) und/oder die sekundäre Beleuchtungseinrichtung (20) zu steuern.
